# EUROPEAN PATENT APPLICATION

(11) **EP 1 136 484 A1**
(43) Date of publication of application: **26.09.2001**
(21) Application number: 00400804.1
(22) Date of filing: 23.03.2000
(51) Int. Cl.: C07D 401/04, C07D 401/14, A61K 31/506, A61P 25/28

(54) **2-(Arylalkylamino)pyrimidone derivatives**

(71) Applicant: SANOFI-SYNTHELABO, 75013 Paris (FR); Mitsubishi-Tokyo Pharmaceuticals, Inc., Chuo-ku, Tokyo 103-8405 (JP)
(72) Inventor: Almario-Garcia, Antonio, 92290 Chatenay-Malabry (FR); Frost, Jonathan Reid, 91320 Wissous (FR); Li, Adrien-Tak, 92260 Fontenay-aux-Roses (FR); Ando, Ryoichi, c/o Mitsubishi-Tokyo Pharm., Inc., Yokohama-shi, Kanagawa 227-0033 (JP); Watanabe, Kazutoshi, Mitsubishi-Tokyo Pharm., Inc., Yokohama-shi, Kanagawa 227-0033 (JP)
(74) Representative: Thouret-Lemaitre, Elisabeth

(57) **Abstract**

A pyrimidone derivative represented by formula (I) or a salt thereof: 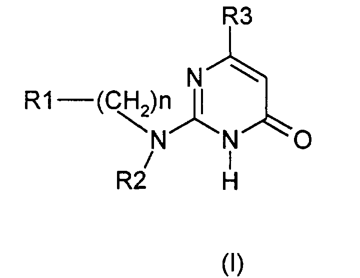 wherein :
When n represents 1 to 5, then R1 represents an unsubstituted napht-1-yl group, an unsubstituted napht-2-yl group or a C_{6,10} aryl group substituted by 1 to 3 substituents selected from a C₁₋₆ alkyl group, halogen atom, a C₁₋₂ perhalogenated alkyl group, a C₁₋₃ halogenated alkyl group, a hydroxyl group, a C₁₋₆ alkoxy group, methylenedioxy group, a nitro, a cyano, an amino, a C₁₋₆ monoalkylamino group, a C₂₋₁₂ dialkylamino group, a (C₁₋₆-alkyl)carbonylamino group, a (C_{6,10}-aryl)carbonylamino group, a (C₁₋₆ -alkoxy)carbonylamino group, aminocarbonyl group, a (C₁₋₆ monoalkylamino)carbonyl group, a (C₂₋₁₂ dialkylamino)carbonyl group, a formyl, a C₁₋₆ alkylcarbonyl group, a (C_{6,10}-aryl)carbonyl group, a C₁₋₆ alkylsulfonyl group, a C_{6,10} arylsulfonyl group, an aminosulfonyl group, a C₁₋₆ monoalkylaminosulfonyl group, a C₂₋₁₂ dialkylaminosulfonyl group, or a phenyl group;the C₁₋₆ alkyl groups and the C₁₋₆ alkoxy groups being optionally substituted; or
When n represents 4 or 5 then R1 can represent an unsubstituted phenyl group;
R2 represents a hydrogen atom or a C₁₋₆ alkyl; and
R3 represents a 2, 3 or 4-pyridyl group optionally substituted by a C₁₋₄ alkyl, group, C₁₋₄ alkoxy group or a halogen atom.

And a medicament comprising the said derivative or a salt thereof as an active ingredient which is used for preventive and/or therapeutic treatment of a neurodegenerative disease caused by abnormal activity of GSK3β.

## Description

### Technical Field

The present invention relates to compounds that are useful as an active ingredient of a medicament for preventive and/or therapeutic treatment of neurodegenerative diseases caused by abnormal activity of GSK3.

### Background Art

GSK3β (glycogen synthase kinase 3β) is a proline directed serine, threonine kinase that plays an important role in the control of metabolism, differentiation and survival. It was initially identified as an enzyme able to phosphorylate and hence inhibit glycogen synthase. It was later recognized that GSK3β was identical to tau protein kinase 1 (TPK1), an enzyme that phosphorylates tau protein in epitopes that are also found to be hyperphosphorylated in Alzheimer's disease and in several taupathies.
Interestingly, protein kinase B (AKT) phosphorylation of GSK3β results in a loss of its kinase activity, and it has been hypothesized that this inhibition may mediate some of the effects of neurotrophic factors. Moreover, phosphorylation by GSK3β of β-catenin, a protein involved in cell survival, results in its degradation by an ubiquitinilation dependent proteasome pathway.
Thus, it appears that inhibition of GSK3β activity may result in neurotrophic activity. Indeed there is evidence that lithium, an uncompetitive inhibitor of GSK3β, enhances neuritogenesis in some models and also increases neuronal survival, through the induction of survival factors such as Bcl-2 and the inhibition of the expression of proapoptotic factors such as P53 and Bax.
Recent studies have demonstrated that β-amyloid increases the GSK3β activity and tau protein phosphorylation. Moreover, this hyperphosphorylation as well as the neurotoxic effects of β-amyloid are blocked by lithium chloride and by a GSK3β antisense mRNA. These observations strongly suggest that GSK3β may be the link between the two major pathological processes in Alzheimer's disease : abnormal APP (Amyloid Precursor protein) processing and tau protein hyperphosphorylation.
Although tau hyperphosphorylation results in a destabilization of the neuronal cytoskeleton, the pathological consequences of abnormal GSK3β activity are, most likely, not only due to a pathological phosphorylation of tau protein because, as mentioned above, an excessive activity of this kinase may affect survival through the modulation of the expression of apoptotic and antiapoptotic factors. Moreover, it has been shown that β-amyloid-induced increase in GSK3β activity results in the phosphorylation and, hence the inhibition of pyruvate dehydrogenase, a pivotal enzyme in energy production and acetylcholine synthesis.

Altogether all these experimental observations indicate that GSK3β may find application in the treatment of the neuropathological consequences and the cognitive and attention deficits associated to Alzheimer's disease, as well as to other acute and chronic neurodegenerative diseases. These include, in a nonlimiting manner, Parkinson's disease, frontoparietal dementia, corticobasal degeneration, Pick's disease, cerebrovascular accidents, peripheral neuropathies and brain and spinal cord trauma.

PCT application WO 98/24782 discloses compounds represented by the following formula (A): 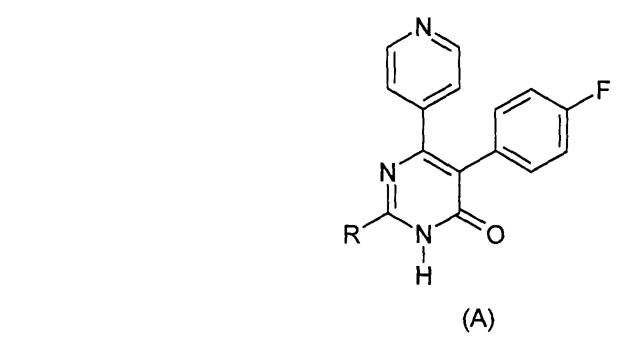 wherein R represents a 2,6-dichlorobenzyl group, a 2-(2-chlorophenyl)ethylamino group, a 3-phenylpropylamino group, or a 1-methyl-3-phenylpropylamino group. The compounds represented by formula (A) are characterized by a 4-fluorophenyl group at the 5-position of the pyrimidine ring. The main pharmacological activity disclosed for the compounds represented by formula (A) is an anti-inflammatory effect, whereas the compounds of the present invention represented by formula (I) herein below are useful as GSK3β inhibitors or as medicaments for the therapeutic treatment of neurodegenerative diseases, and therefore, their pharmacological activities are totally different.

### Disclosure of the Invention

An object of the present invention is to provide compounds useful as an active ingredient of a medicament for preventive and/or therapeutic treatment of neurodegenerative diseases. More specifically, the object is to provide novel compounds useful as an active ingredient of a medicament that enables prevention and/or treatment of the neurodegenerative diseases such as Alzheimer's disease.
Thus, the inventors of the present invention have identified compounds possessing inhibitory activity against GSK3β. As a result, they found that compounds represented by the following formula (I) had the desired activity and were useful as an active ingredient of a medicament for preventive and/or therapeutic treatment of the aforementioned diseases.

The present invention thus provides pyrimidone derivatives represented by formula (I) or salts thereof, solvates thereof or hydrates thereof: 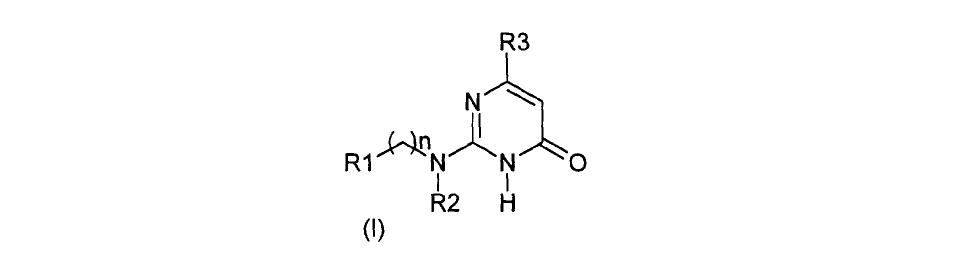 wherein:
When n represents 1 to 5, then R1 represents an unsubstituted napht-1-yl group, an unsubstituted napht-2-yl group or a C_{6,10} aryl group substituted by 1 to 3 substituents selected from a C₁₋₆ alkyl group, halogen atom, a C₁₋₂ perhalogenated alkyl group, a C₁₋₃ halogenated alkyl group, a hydroxyl group, a C₁₋₆ alkoxy group, methylenedioxy group, a nitro, a cyano, an amino, a C₁₋₆ monoalkylamino group, a C₂₋₁₂ dialkylamino group, a (C₁₋₆-alkyl)carbonylamino group, a (C_{6,10}-aryl)carbonylamino group, a (C₁₋₆ -alkoxy)carbonylamino group, aminocarbonyl group, a (C₁₋₆ monoalkylamino)carbonyl group, a (C₂₋₁₂ dialkylamino)carbonyl group, a formyl, a C₁₋₆ alkylcarbonyl group, a (C_{6,10}-aryl)carbonyl group, a C₁₋₆ alkylsulfonyl group, a C_{6,10} arylsulfonyl group, an aminosulfonyl group, a C₁₋₆ monoalkylaminosulfonyl group, a C₂₋₁₂ dialkylaminosulfonyl group, or a phenyl group;
   the C₁₋₆ alkyl groups and the C₁₋₆ alkoxy groups being optionally substituted by a halogen atom, a hydroxyl group, a C₁₋₆ alkoxy group, an amino, a C₁₋₆ monoalkylamino group, a C₂₋₁₂ dialkylamino group, a (C₁₋₆alkyl)carbonylamino group, a (C_{6,10} aryl)carbonylamino group, a (C₁₋₆ alkoxy)carbonylamino group, a C₁₋₆ alkylsulfonylamino group, a C_{6,10} arylsulfonylamino group, a phenyl group, a pyridine, a pyrimidine, or a pyrimidin-2-yl-amino, or
When n represents 4 or 5 then R1 can represent an unsubstituted phenyl group;
R2 represents a hydrogen atom or a C₁₋₆ alkyl; and
R3 represents a 2, 3 or 4-pyridyl group optionally substituted by a C₁₋₄ alkyl, group, C₁₋₄ alkoxy group or a halogen atom.

According to another aspect of the present invention, there is provided a medicament comprising as an active ingredient a substance selected from the group consisting of the pyrimidone derivatives represented by formula (I) and the physiologically acceptable salts thereof, and the solvates thereof and the hydrates thereof. As preferred embodiments of the medicament, there are provided the aforementioned medicament which is used for preventive and/or therapeutic treatment of diseases caused by abnormal GSK3β activity, and the aforementioned medicament which is used for preventive and/or therapeutic treatment of neurodegenerative diseases. As further preferred embodiments of the present invention, there are provided the aforementioned medicament wherein the diseases are selected from the group consisting of Alzheimer's disease, Parkinson's disease, frontoparietal dementia, corticobasal degeneration, Pick's disease, cerebrovascular accidents, brain and spinal cord trauma, and peripheral neuropathies; and the aforementioned medicament in the form of pharmaceutical composition containing the above substance as an active ingredient together with one or more pharmaceutical additives.

The present invention further provides an inhibitor of GSK3β activity comprising as an active ingredient a substance selected from the group consisting of the pyrimidone derivatives of formula (I) and the salts thereof, and the solvates thereof and the hydrates thereof.

According to further aspects of the present invention, there are provided a method for preventive and/or therapeutic treatment of neurodegenerative diseases caused by abnormal GSK3β activity, which comprises the step of administering to a patient a preventively and/or therapeutically effective amount of a substance selected from the group consisting of the pyrimidone derivatives of formula (I) and the physiologically acceptable salts thereof, and the solvates thereof and the hydrates thereof; and a use of a substance selected from the group consisting of the pyrimidone derivatives of formula (I) and the physiologically acceptable salts thereof, and the solvates thereof and the hydrates thereof for the manufacture of the aforementioned medicament.

As used herein, the C ₁₋₆ alkyl group represents a straight or branched alkyl group having 1 to 6 carbon atoms, for example, methyl group, ethyl group, n-propyl group, isopropyl group, n-butyl group, isobutyl group, sec-butyl group, tert-butyl group, n-pentyl group, isopentyl group, neopentyl group, 1,1-dimethylpropyl group, n-hexyl group, isohexyl group, and the like;
The C_{6,10} aryl group represents a phenyl group, a napht-1-yl group or a napht-2-yl group;
The C ₁₋₆ alkoxy group represents an alkyl-oxy for example, methoxy group, ethoxy group, propoxy group, isopropoxy group, butoxy group, isobutoxy group, sec-butoxy group, tert-butoxy group, pentyloxy group, isopentyloxy group, neopentyloxy group, 1,1-dimethylpropyloxy group;
The halogen atom represents a fluorine, chlorine, bromine or iodine atom;
The C₁₋₂ perhalogenated alkyl group represents an alkyl group wherein all the hydrogen have been subsituted by a halogeno, for example a CF₃ or C₂F₅,
The C₁₋₃ halogenated alkyl group represents an alkyl group wherein at least one hydrogen has not been subsituted by a halogeno,
The C₁₋₆ monoalkylamino group represents an amino substituted by one C₁₋₆ alkyl group, for example, methylamino group, ethylamino group, propylamino group, isopropylamino group, butylamino group, isobutylamino group, tert-butylamino group, pentylamino group and isopentylamino group;
The C₂₋₁₂ dialkylamino group represents an amino substituted by two C₁₋₆ alkyl groups, for example, dimethylamino group, ethylmethylamino group, diethylamino group, methylpropylamino group and diisopropylamino group;
The (C₁₋₆ alkyl)carbonylamino group represents an amino group substituted by a C₁₋₆ acyl group, for example, formyl group, acetyl group, propionyl group, pivaloyl group, butyryl group, isobutyryl group, pentanoyl group, 3-methylbutyryl group, hexanoyl group;
The (C_{6,10} aryl)carbonylamino group represents an amino group substituted by a benzoyl group or a naphthylenecarbonyl group;
The (C₁₋₆ alkoxy)carbonylamino group represents an amino group substituted with a (C₁₋₆ alkoxy)carbonyl group, such as for example, methoxycarbonyl group, ethoxycarbonyl group, propoxycarbonyl group, *tert*-butoxycarbonyl group, pentyloxycarbonyl group, hexyloxycarbonyl group;
The C₁₋₆ monoalkylaminocarbonyl group represents an aminocarbonyl group substituted by one C₁₋₆ alkyl group, as defined and illustrated above, for example : methylaminocarbonyl group, ethylaminocarbonyl group, propylaminocarbonyl group, *i*-propylaminocarbonyl group, butylaminocarbonyl group, *iso*-butylaminocarbonyl group, *tert*-butylaminocarbonyl group, pentylaminocarbonyl group, neopentylaminocarbony group, 1,1-dimethylpropylaminocarbonyl group, n-hexylaminocarbonyl group, isohexylaminocarbonyl group, and the like;
The C₂₋₁₂ dialkylaminocarbonyl group represents an aminocarbonyl group substituted by two C₁₋₆ alkyl groups, as defined and illustrated above, for example : dimethylaminocarbonyl group, diethylaminocarbonyl group, dipropylaminocarbonyl group, di-*i*-propylaminocarbonyl group, butylaminocarbonyl group, *iso*-butylaminocarbonyl group, *tert*-butylaminocarbonyl group, dipentylaminocarbonyl group, di-neopentylaminocarbony group, di-(1,1-dimethylpropyl)aminocarbonyl group, di-n-hexylaminocarbonyl group, di-isohexylaminocarbonyl group, ethylmethylaminocarbonyl group, ethylpropylaminocarbonyl group, ethyl-*tert*-butylaminocarbonyl group, and the like;
The C₁₋₆ alkylcarbonyl group represents an acyl group having 1 to 6 carbon atoms, such as, for example, formyl group, acetyl group, propionyl group, pivaloyl group, butyryl group, isobutyryl group, pentanoyl group, 3-methylbutyryl group, hexanoyl group;
The (C_{6,10}-aryl)carbonyl group represents an arylcarbonyl group wherein the C_{6,10} aryl group is as defined here above, such as, for example benzoyl group and a naphthylenecarbonyl group.
The C₁₋₆ alkylsulfonyl group represents an alkylsulfonyl group having 1 to 6 carbon atoms, such as, for example, methylsulfonyl group, ethylsulfonyl group, n-propylsulfonyl group, isopropylsulfonyl group, n-butyl group, isobutylsulfonyl group, sec-butylsulfonyl group, tert-butylsulfonyl group, n-pentylsulfonyl group, isopentylsulfonyl group, neopentylsulfonyl group, 1,1-dimethylpropylsulfonyl group, n-hexylsulfonyl group, isohexylsulfonyl group, and the like;
The C_{6,10} arylsulfonyl group represents an arylsulfonyl group wherein the C_{6,10} aryl group is as defined here above, such as, for example, phenylsulfonyl group or naphthalenesulfonyl group;
The C₁₋₆ monoalkylaminosulfonyl group represents an aminosulfonyl group substituted by one C₁₋₆ alkyl group, as defined and illustrated above, for example : methylaminosulfonyl group, ethylaminosulfonyll group, propylaminosulfonyl group, *i*-propylaminocarbonyl group, butylaminosulfonyl group, iso-butylaminosulfonyl group, *tert*-butylaminosulfonyl group, 1,1-dimethylpropylaminosulfonyl group, n-hexylaminosulfonyl group, isohexylaminosulfonyl group, and the like;
The C₂₋₁₂ dialkylaminosulfonyl group represents a aminosulfonyl group substituted by two C₁₋₆ alkyl group, as defined and illustrated above, for example : dimethylaminosulfonyl group, diethylaminosulfonyll group, dipropylaminosulfonyl group, *i*-propylaminocarbonyl group, butylaminosulfonyl group, *iso*-dibutylaminosulfonyl group, di-*tert*-butylaminosulfonyl group, di-(1,1-dimethylpropyl)aminosulfonyl group, di-n-hexylaminosulfonyl group, di-isohexylaminosulfonyl group, ethylmethylaminocarbonyl group, ethylpropylaminocarbonyl group, ethyl-*tert*-butylaminocarbonyl group, and the like.

The compounds represented by the aforementioned formula (I) may form a salt. Examples of the salt include, when an acidic group exists, salts of alkali metals and alkaline earth metals such as lithium, sodium, potassium, magnesium, and calcium; salts of ammonia and amines such as methylamine, dimethylamine, trimethylamine, dicyclohexylamine, tris(hydroxymethyl)aminomethane, N,N-bis(hydroxyethyl)piperazine, 2-amino-2-methyl-1-propanol, ethanolamine, N-methylglucamine, and L-glucamine; or salts with basic amino acids such as lysine, δ-hydroxylysine, and arginine. The base-addition salts of acidic compounds are prepared by standard procedures well known in the art.

When a basic group exists, examples include salts with mineral acids such as hydrochloric acid, hydrobromic acid, sulfuric acid, nitric acid, phosphoric acid; salts with organic acids such as methanesulfonic acid, benzenesulfonic acid, p-toluenesulfonic acid, acetic acid, propionic acid, tartaric acid, fumaric acid, maleic acid, malic acid, oxalic acid, succinic acid, citric acid, benzoic acid, mandelic acid, cinnamic acid, lactic acid, glycolic acid, glucuronic acid, ascorbic acid, nicotinic acid, and salicylic acid; or salts with acidic amino acids such as aspartic acid, and glutamic acid.

The acid-addition salts of the basic compounds are prepared by standard procedures well know in the art which include, but are not limited thereto, dissolving the free base in an aqueous alcohol solution containing the appropriate acid and isolating the salt by evaporating the solution, or by reacting the free base and an acid in an organic solvent, in which case the salt separates directly, or is precipitated with a second organic solvent, or can be obtained by concentration of the solution. The acids which can be used to prepare the acid-addition salts include preferably those which produce, when combined with the free base, pharmaceutically-acceptable salts, that is, salts whose anions are relatively innocuous to the animal organism in pharmaceutical doses of the salts, so that the beneficial properties inherent in the free base are not compromised by side effects ascribable to the anions. Although medicinally acceptable salts of the basic compounds are preferred, all acid-addition salts are within the scope of the present invention.

In addition to the pyrimidone derivatives represented by the aforementioned formula (I) and salts thereof, their solvates and hydrates also fall within the scope of the present invention. The pyrimidone derivatives represented by the aforementioned formula (I) may have one or more asymmetric carbon atoms. As for the stereochemistry of such asymmetric carbon atoms, they may independently be in either (R) and (S) configuration, and the pyrimidone derivative may exist as stereoisomers such as optical isomers, or diastereoisomers. Any stereoisomers in pure form, any mixtures of stereoisomers, racemates and the like fall within the scope of the present invention.

Furthermore, as the pyrimidone derivatives represented by the aforementioned formula (I), a 3H-4-one compound, a 4-hydroxy compound, and a 1H-4-one compound may exist as tautomers. The existence of such tautomers is readily apparent to those skilled in the art, and these tautomers fall within the scope of the present invention.

Examples of preferred compounds of the present invention are shown in table 1 thereafter. However, the scope of the present invention is not limited by these compounds.

Preferred compounds of the present invention represented by formula (I) include also compounds wherein R3 represents a 3- or 4-pyridyl group and more preferably 4-pyridyl group, which may be substituted by a C₁₋₂ alkyl, group, C₁₋₂ alkoxy group or a halogen atom. More preferred compounds of the present invention represented by formula (I) include also
(1) Compounds wherein R2 represents a 4-pyridyl group which is unsubstituted;
(2) Compounds wherein n represents 1 to 4.

Particularly preferred compounds of the present invention represented by formula (I) include :
2-[[(3,4-dimethoxyphenyl)methyl]amino]-6-pyridin-4-ylpyrimidin-4-(1H)-one,
2-[[2-(3,4-dimethoxyphenyl)ethyl]methylamino]-6-pyridin-4-ylpyrimidin-4-(1H)-one,
2-[[2-(4-methoxyphenyl)ethyl]amino]-6-pyridin-4-ylpyrimidin-4-(1H)-one,
2-[[2-(3-methoxyphenyl)ethyl]amino]-6-pyridin-4-ylpyrimidin-4-(1H)-one,
2-[[2-(2-methoxyphenyl)ethyl]amino]-6-pyridin-4-ylpyrimidin-4-(1H)-one,
2-[[2-(2-fluorophenyl)ethyl]amino]-6-pyridin-4-ylpyrimidin-4-(1H)-one,
2-[[2-(3-fluorophenyl)ethyl]amino]-6-pyridin-4-ylpyrimidin-4-(1H)-one,
2-[[2-(4-fluorophenyl)ethyl]amino]-6-pyridin-4-ylpyrimidin-4-(1H)-one,
2-[[2-(4-bromophenyl)ethyl]amino]-6-pyridin-4-ylpyrimidin-4-(1H)-one,
2-[[2-(2,4-dichlorophenyl)ethyl]amino]-6-pyridin-4-ylpyrimidin-4-(1H)-one,
2-[[2-(2-chlorophenyl)ethyl]amino]-6-pyridin-4-ylpyrimidin-4-(1H)-one,
2-[[2-(4-chlorophenyl)ethyl]amino]-6-pyridin-4-ylpyrimidin-4-(1H)-one,
2-[[2-(4-nitrophenyl)ethyl]amino]-6-pyridin-4-ylpyrimidin-4-(1H)-one,
2-[[2-(4-aminophenyl)ethyl]amino]-6-pyridin-4-ylpyrimidin-4-(1H)-one,
2-[[2-(3,4-dimethoxyphenyl)ethyl]amino]-6-pyridin-4-ylpyrimidin-4-(1H)-one,
2-[[2-(2,5-dimethoxyphenyl)ethyl]amino]-6-pyridin-4-ylpyrimidin-4-(1H)-one,
2-[[2-(4-hydroxyphenyl)ethyl]amino]-6-pyridin-4-ylpyrimidin-4-(1H)-one,
2-[[2-(4-methylphenyl)ethyl]amino]-6-pyridin-4-ylpyrimidin-4-(1H)-one,
2-[[2-(4-aminosulfonylphenyl)ethyl]amino]-6-pyridin-4-ylpyrimidin-4-(1H)-one,
2-[[2-(3-chlorophenyl)ethyl)amino)-6-pyridin-4-ylpyrimidin-4-(1H)-one,
2-[[4-(phenyl)butyl]amino]-6-pyridin-4-ylpyrimidin-4-(1H)-one,
2-[[2-(4-phenylphenyl)ethyl]amino]-6-pyridin-4-ylpyrimidin-4-(1H)-one,
2-[[2-(2-naphthyl)ethyl]amino]-6-pyridin-4-ylpyrimidin-4-(1H)-one,
2-[[[3-(aminomethyl)phenyl]methyl]amino]-6-pyridin-4-ylpyrimidin-4-(1H)-one,
2-[[[4-(aminomethyl)phenyl]methyl]amino]-6-pyridin-4-ylpyrimidin-4-(1H)-one,
2-[[(3-methylphenyl)methyl]amino]-6-pyridin-4-ylpyrimidin-4-(1H)-one,
2-[[(4-methoxyphenyl)methyl]amino]-6-pyridin-4-ylpyrimidin-4-(1H)-one,
2-[[(4-fluorophenyl)methyl]amino]-6-pyridin-4-ylpyrimidin-4-(1H)-one,
2-[[(2-chlorophenyl)methyl]amino]-6-pyridin-4-ylpyrimidin-4-(1H)-one,
2-[[(4-chlorophenyl)methyl]amino]-6-pyridin-4-ylpyrimidin-4-(1H)-one,
2-[[[4-(trifluoromethyl)phenyl]methyl]amino]-6-pyridin-4-ylpyrimidin-4-(1H)-one,
2-[[[4-(3-aminopropoxy)phenyl]methyl]amino]-6-pyridin-4-ylpyrimidin-4-(1H)-one,
2-[[(3,4-dimethoxyphenyl)methyl]amino]-6-pyridin-4-ylpyrimidin-4-(1H)-one,
2-[[(3-nitrophenyl)methyl]amino]-6-pyridin-4-ylpyrimidin-4-(1H)-one,
2-[[(2-aminophenyl)methyl]amino]-6-pyridin-4-ylpyrimidin-4-(1H)-one,
2-[[(2-methylphenyl)methyl]amino]-6-pyridin-4-ylpyrimidin-4-(1H)-one,
2-[[(4-methylphenyl)methyl]amino]-6-pyridin-4-ylpyrimidin-4-(1H)-one,
2-[[(2-methoxyphenyl)methyl]amino]-6-pyridin-4-ylpyrimidin-4-(1H)-one,
2-[[(3-methoxyphenyl)methyl]amino]-6-pyridin-4-ylpyrimidin-4-(1H)-one,
2-[[(3-chlorophenyl)methyl]amino]-6-pyridin-4-ylpyrimidin-4-(1H)-one,
2-[[(4-aminophenyl)methyl]amino]-6-pyridin-4-ylpyrimidin-4-(1H)-one,
2-[[[3-(acetamidomethyl)phenyl]methyl]amino]-6-pyridin-4-ylpyrimidin-4-(1H)-one,
2-[[[3-(3-aminopropoxy)phenyl]methyl]amino]-6-pyridin-4-ylpyrimidin-4-(1H)-one,
2-[[[3-[(pyridin-2-yl)methoxy]phenyl]methyl]amino]-6-pyridin-4-ylpyrimidin-4-(1H)one,
2-[[[3-[3-(pyridin-3-yl)propoxy]phenyl]methyl]amino]-6-pyridin-4-ylpyrimidin-4-(1H)one,
2-[[[3-(*tert*-butyloxycarbonylaminomethyl)phenyl]methyl]amino]-6-pyridin-4-ylpyrimidin-4-(1H)-one,
2-[[(3-aminophenyl)methyl]amino]-6-pyridin-4-ylpyrimidin-4-(1H)-one,
2-[[[3-(benzoylaminomethyl)phenyl]methyl]amino]-6-pyridin-4-ylpyrimidin-4-(1H)one,
2-[[[4-(2-aminoethoxy)phenyl]methyl]amino]-6-pyridin-4-ylpyrimidin-4-(1H)-one,
2-[[[3-(methanesulfonylaminomethyl)phenyl]methyl]amino]-6-pyridin-4-ylpyrimidin-4-(1H)-one,
2-[[[3-[(pyrimidin-2-yl)aminomethyl]phenyl]methyl]amino]-6-pyridin-4-ylpyrimidin-4-(1H)-one,
2-[[[3-(n-butylaminomethyl)phenyl]methyl]amino]-6-pyridin-4-ylpyrimidin-4-(1H)-one,
2-[[[3-(2-aminoethoxy)phenyl]methyl]amino]-6-pyridin-4-ylpyrimidin-4-(1H)-one,
2-[[[3-(4-aminobutoxy)phenyl]methyl]amino]-6-pyridin-4-ylpyrimidin-4-(1H)-one,
2-[[3-(2-methylphenyl)propyl]amino]-6-pyridin-4-ylpyrimidin-4-(1H)-one,
2-[[3-(3-methylphenyl)propyl]amino]-6-pyridin-4-ylpyrimidin-4-(1H)-one,
2-[[3-()propyl]amino]-6-pyridin-4-ylpyrimidin-4-(1H)-one,
2-[[3-(2-methoxyphenyl)propyl]amino]-6-pyridin-4-ylpyrimidin-4-(1H)-one,
2-[[3-(3-methoxyphenyl)propyl]amino]-6-pyridin-4-ylpyrimidin-4-(1H)-one,
2-[[3-(4-methoxyphenyl)propyl]amino]-6-pyridin-4-ylpyrimidin-4-(1H)-one,
2-[[3-(2-chlorophenyl)propyl]amino]-6-pyridin-4-ylpyrimidin-4-(1H)-one,
2-[[3-(3-chlorophenyl)propyl]amino]-6-pyridin-4-ylpyrimidin-4-(1H)-one,
2-[[3-(4-chlorophenyl)propyl]amino]-6-pyridin-4-ylpyrimidin-4-(1H)-one,
2-[[[3-[3-(pyridin-4-yl)propoxy]phenyl]methyl]amino]-6-pyridin-4-ylpyrimidin-4-(1H)one,
2-[[[3-[(pyridin-3-yl)methoxy]phenyl]methyl]amino]-6-pyridin-4-ylpyrimidin-4-(1H)one,
2-[[[3-[2-(pyridin-2-yl)ethoxy]phenyl]methyl]amino]-6-pyridin-4-ylpyrimidin-4-(1H)one,
2-[[[3-(*tert*-butyloxycarbonylaminomethyl)phenyl]methyl]methylamino]-6-pyridin-4-ylpyrimidin-4-(1H)-one,
2-[[[3-(methylamino)phenyl]methyl]methylamino]-6-pyridin-4-ylpyrimidin-4-(1H)one,
2-[[3-(3,4-dimethoxyphenyl)]propyl]amino]-6-pyridin-4-ylpyrimidin-4-(1H)-one, and
2-[[3-(4-phenylphenyl)]propyl]amino]-6-pyridin-4-ylpyrimidin-4-(1H)-one.

As a further object, the present invention concerns also methods for preparing the pyrimidone compounds represented by the aforementioned formula (I).

These compounds can be prepared, for example, according to methods explained below.

### 1. Preparation Method 1

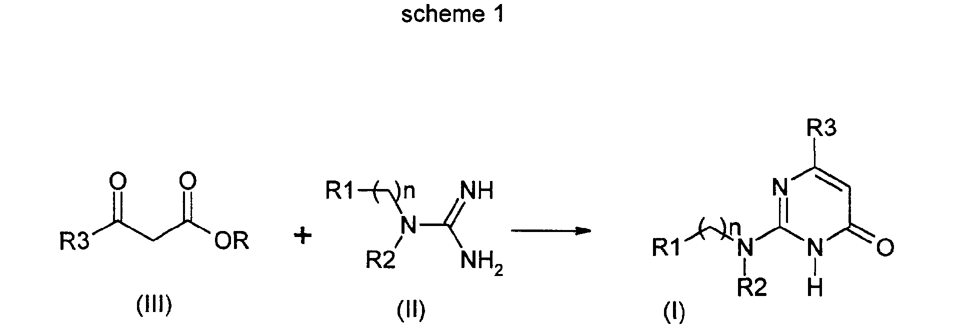

(In the above scheme, R represents an alkyl group, which may be substituted, and definitions of R1,R2, R3, and n are the same as those already described for compound of formula (I).)

The 3-ketoester represented by the above formula (III) is allowed to react with the compound represented by formula (II) or a salt thereof to obtain the compound of the aforementioned formula (I) in the presence of a base such as lithium tert-butoxide, sodium tert-butoxide, potassium tert-butoxide, lithium methoxide, sodium methoxide, potassium methoxide, lithium ethoxide, sodium ethoxide, potassium ethoxide, 1,8-diazabicyclo[5,4,0]undec-7-ene, triethylamine, diisopropylethylamine, dimethylbenzylamine, dimethylaniline, diethylaniline and the like.

Examples of a solvent suitable for the reaction include, for example, alcoholic solvent such as methanol, ethanol, 1-propanol, isopropanol, tert-butanol; etheric solvents such as diethyl ether, tert-butyl methyl ether, tetrahydrofuran, isopropyl ether; hydrocarbon solvents such as benzene, toluene, xylene; halogenated solvents such as dichloromethane, chloroform, dichloroethane; aprotic polar solvents such as formamide, N,N-dimethylformamide, N,N-dimethylacetamide, N-methylpyrrolidone, dimethyl sulfoxide, sulfolane, hexamethylphosphoric triamide and the like. Generally, a single solvent or a mixture of two or more solvents may be used depending on the base used, and the reaction may be carried out for 1 hour to 14 days at a suitable temperature ranging from 0° to 250°C under nitrogen or argon atmosphere or in ordinary air.

Compounds of formula (III) and formula (II) are commercially available or may be synthesized according to known methods of one skilled in the art. For example compounds of formula (III), wherein R3 represent a 4-pyridyl group optionally substituted by a C₁₋₄ alkyl group, C₁₋₄ alkoxy group or a halogen atom, can be prepared by reacting a nicotinic acid optionally substituted by a C₁₋₄ alkyl group, C₁₋₄ alkoxy group or an halogen, with a malonic acid monoester. The reaction can be carried out using methods well known to one skilled in the art, such as for example in presence of a coupling agent such as 1,1'-carbonylbis-1H-imidazole in a solvent such as a tetrahydrofuran at a temperature ranging from 20 to 70°C.

### 2. Preparation method 2

Alternatively pyrimidone compounds represented by the aforementioned formula (I) may be prepared according to scheme 2. 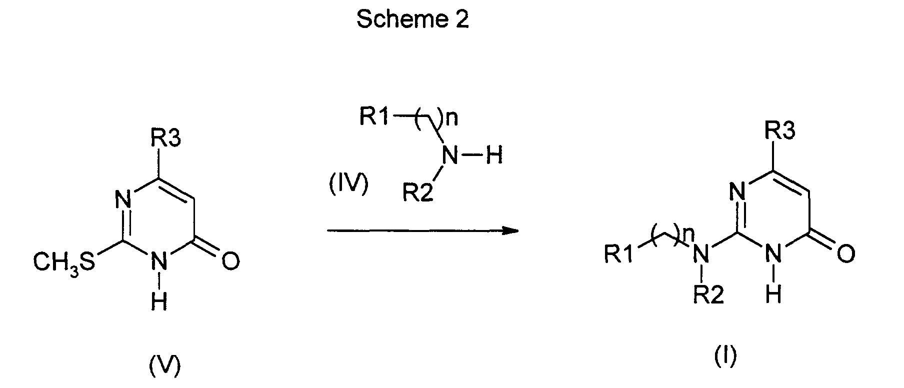 (In the above scheme the definition of R1, R2, R3 and n are the same as those already described.)

The 2-methylthio derivative represented by the above formula (V), wherein R3 is as defined for compound of formula (I), is allowed to react with an amine of formula (IV) to obtain the compound of the aforementioned formula (I). The reaction may be carried out in a solvent such as, for example, an alcoholic solvent such as n-pentanol or isoamyl alcohol at a suitable temperature ranging from 100 to 180 °C under ordinary air.

Compound of formula (V) may be prepared according to the method defined in scheme 3. 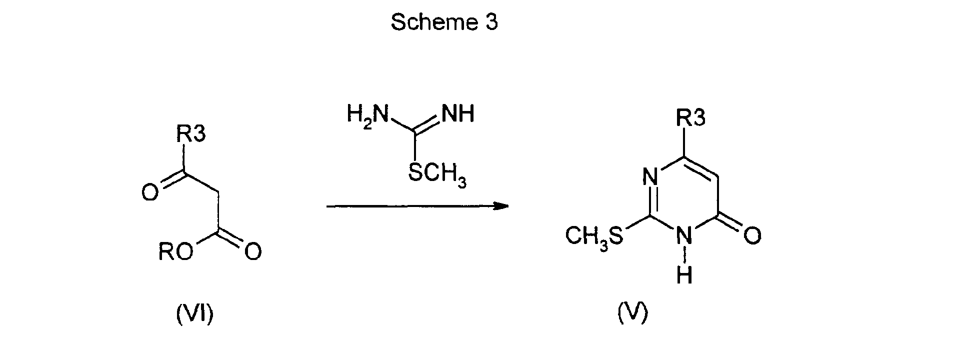 (In the above scheme the definition of R3 is the same as already described.)

According to this method, the 3-ketoester of formula (VI) is allowed to react with a 2-methyl-2-thiopseudourea sulfate. The reaction may be carried out in solvent such as water or an alcohol, such as ethanol, propanol and butanol, at a suitable temperature ranging from 25-100°C under ordinary air.

Compounds of formula (IV) are commercially available or may be synthesized according to well-known methods of one skilled in the art.

In addition when applicable, compound of formula (I) could be derivatised into other compound of formula (I), using well known methods in the art. This is the case, for example, R1 or a substituent on an alkyl or alkoxy group, could be oxidized, hydrogenated, alkylated..., or be transformed using well known method in the art to give another R1 group or a substituent within the scope of the present invention.

In the above reactions, protection or deprotection of a functional group may sometimes be necessary. A suitable protecting group can be chosen depending on the type of a functional group, and a method described in the literature may be applied. Examples of protecting groups, of protection and deprotection methods are given for example in *Protective groups in Organic Synthesis* Greene et al., 2nd Ed. (John Wiley & Sons, Inc., New York).

The compounds of the present invention have inhibitory activity against GSK3β. Accordingly, the compounds of the present invention are useful as an active ingredient for the preparation of a medicament, which enables preventive and/or therapeutic treatment of neurodegenerative diseases such as Alzheimer's disease. In addition, the compounds of the present invention are also useful as an active ingredient for the preparation of a medicament for preventive and/or therapeutic treatment of Parkinson's disease, frontoparietal dementia, corticobasal degeneration, Pick's disease, cerebrovascular accidents, brain and spinal cord trauma, and peripheral neuropathies.

The present invention further relates to a method for treating neurodegenerative diseases caused by abnormal activity of GSK3β and of the aforementioned diseases which comprises administrating to a mammalian organism in need thereof an effective amount of a compound of the formula (I).

As the active ingredient of the medicament of the present invention, a substance may be used which is selected from the group consisting of the compound represented by the aforementioned formula (I) and pharmacologically acceptable salts thereof, and solvates thereof and hydrates thereof. The substance, per se, may be administered as the medicament of the present invention, however, it is desirable to administer the medicament in a form of a pharmaceutical composition which comprises the aforementioned substance as an active ingredient and one or more of pharmaceutical additives. As the active ingredient of the medicament of the present invention, two or more of the aforementioned substances may be used in combination. The above pharmaceutical composition may be supplemented with an active ingredient of another medicament for the treatment of the above mentioned diseases. A type of the pharmaceutical composition is not particularly limited, and the composition may be provided as any formulation for oral or parenteral administration. For example, the pharmaceutical composition may be formulated, for example, in the form of pharmaceutical compositions for oral administration such as granules, fine granules, powders, hard capsules, soft capsules, syrups, emulsions, suspensions, solutions and the like, or in the form of pharmaceutical compositions for parenteral administrations such as injections for intravenous, intramuscular, or subcutaneous administration, drip infusions, transdermal preparations, transmucosal preparations, nasal drops, inhalants, suppositories and the like. Injections or drip infusions may be prepared as powdery preparations such as in the form of lyophilized preparations, and may be used by dissolving just before use in an appropriate aqueous medium such as physiological saline. Sustained-release preparations such as those coated with a polymer may be directly administered intracerebrally.

Types of pharmaceutical additives used for the manufacture of the pharmaceutical composition, content ratios of the pharmaceutical additives relative to the active ingredient, and methods for preparing the pharmaceutical composition may be appropriately chosen by those skilled in the art. Inorganic or organic substances, or solid or liquid substances may be used as pharmaceutical additives. Generally, the pharmaceutical additives may be incorporated in a ratio ranging from 1% by weight to 90% by weight based on the weight of an active ingredient.
Examples of excipients used for the preparation of solid pharmaceutical compositions include, for example, lactose, sucrose, starch, talc, cellulose, dextrin, kaolin, calcium carbonate and the like. For the preparation of liquid compositions for oral administration, a conventional inert diluent such as water or a vegetable oil may be used. The liquid composition may contain, in addition to the inert diluent, auxiliaries such as moistening agents, suspension aids, sweeteners, aromatics, colorants, and preservatives. The liquid composition may be filled in capsules made of an absorbable material such as gelatin. Examples of solvents or suspension mediums used for the preparation of compositions for parenteral administration, e.g. injections, suppositories, include water, propylene glycol, polyethylene glycol, benzyl alcohol, ethyl oleate, lecithin and the like. Examples of base materials used for suppositories include, for example, cacao butter, emulsified cacao butter, lauric lipid, witepsol.

Dose and frequency of administration of the medicament of the present invention are not particularly limited, and they may be appropriately chosen depending on conditions such as a purpose of preventive and/or therapeutic treatment, a type of a disease, the body weight or age of a patient, severity of a disease and the like. Generally, a daily dose for oral administration to an adult may be 0.01 to 1,000 mg (the weight of an active ingredient), and the dose may be administered once a day or several times a day as divided portions, or once in several days. When the medicament is used as an injection, administrations may preferably be performed continuously or intermittently in a daily dose of 0.001 to 100 mg (the weight of an active ingredient) to an adult.

### Chemical Examples

The present invention will be explained more specifically with reference to the following general examples, however, the scope of the present invention is not limited to these examples.

### Example 1: preparation of 2-(arylalkylamino)-6-pyridin-4-ylpyrimidin-4(1H)-one.

### 1.1. Preparation of Ethyl 3-(4-pyridyl)-3-oxopropionate

Isonicotinic acid (35.56 g, 289 mmol) was added to a solution of 1,1'-carbonylbis-1H-imidazole (46.98 g, 290 mmol) in tetrahydrofuran (700ml), and the resulting solution was stirred for 1.5 hr at 50°C. After cooling to room temperature, malonic acid monoester potassium salt (51.7 g, 304 mmol) and magnesium chloride (34.33 g, 361 mmol) were added, and the mixture was refluxed for 1 hr and then heated at 50°C for 6 hr. The solvent was removed under reduced pressure and the residue was quenched by the addition of dilute acetic acid. The organic layer was extracted with ethyl acetate (3 times) and the combined extracts were washed with dilute aqueous sodium bicarbonate and brine, and were concentrated under reduced pressure. The residue was purified by silica gel column chromatography (eluent; hexane/ethyl acetate = 2/1 to 1/1) and recrystallization from hexane - ethyl acetate gave 41.52 g (74%) of the title compound.

### 1.2. Preparation of 2-(arylalkylamino)-6-pyridin-4-ylpyrimidin-4(1H)-one.

Ethyl 3-(4-pyridyl)-3-oxopropionate (0.60g ; 3.1mmol), 1-amidino-4-(R1 substitutent)piperazine hydrochloride (3.4 mmol) and potassium carbonate (1.15g ; 8.3mmol) were added to 5 ml of ethanol, and the mixture was heated under reflux at 75° for 20 hours. Acetic acid was added to the reaction mixture , and the solvent was removed by distillation. The residue was treated with water and then with acetic acid , and the resulting solid was separated by filtration, washed with water and ethyl acetate, and dried to obtain the desired compound.

### 2. Example 2 : Preparation of 2-(arylalkylamino)-6-pyridin-4-ylpyrimidin-4(1H)-one.

### 2.1. Preparation of 2-(Methylthio)-6-pyridinyl-4-ylpyrimidin-4(1H)-one

To a solution of 5.76 g (20.7 mmol) of 2-methyl-2-thiopseudoiurea sulfate in 48 ml of water was added 4.85 g (86.52 mmol) of potassium hydroxide. The mixture was agitated and 8.0 g (41.4 mmol) of ethyl 3-(4-pyridyl)-3-oxopropionate was added and stirring was maintained for 48 hours.
The precipitate was recovered by filtration and was washed with water and then ether. The product was dried at 90°C in vacuo to give 6.26 g, 69% of white solid.
Mp : 328-330°C.

### 2.2 Preparation of 2-(arylalkylamino)-6-pyridin-4-ylpyrimidin-4(1 H)-one

A solution of 1 equivalent of 2-(methylthio)-6-pyridinyl-4-ylpyrimidin-4(1H)-one and 3 to 5 equivalents of a piperazine of formula (V) were suspended in amyl alcohol (0.1 - 0.2 M) and was heated at 150°C during 72 hours.The cooled solution was concentrated and purified by chromatography on silica gel.

Table 1 : a list of chemical structures and physical data for compounds of the aforementioned formula (I) illustrating the present invention is given in table 1.

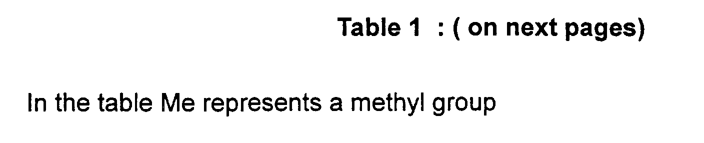

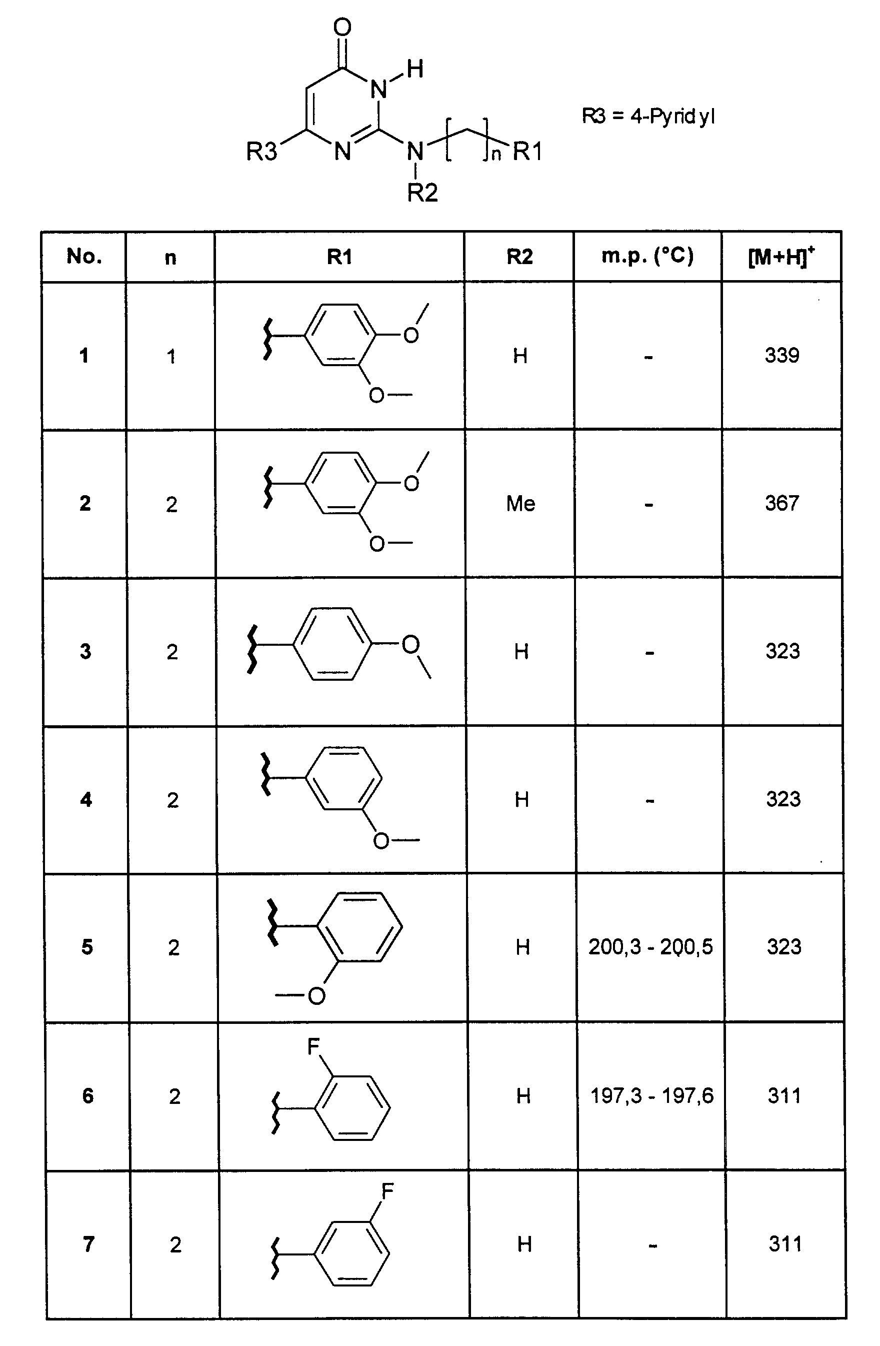

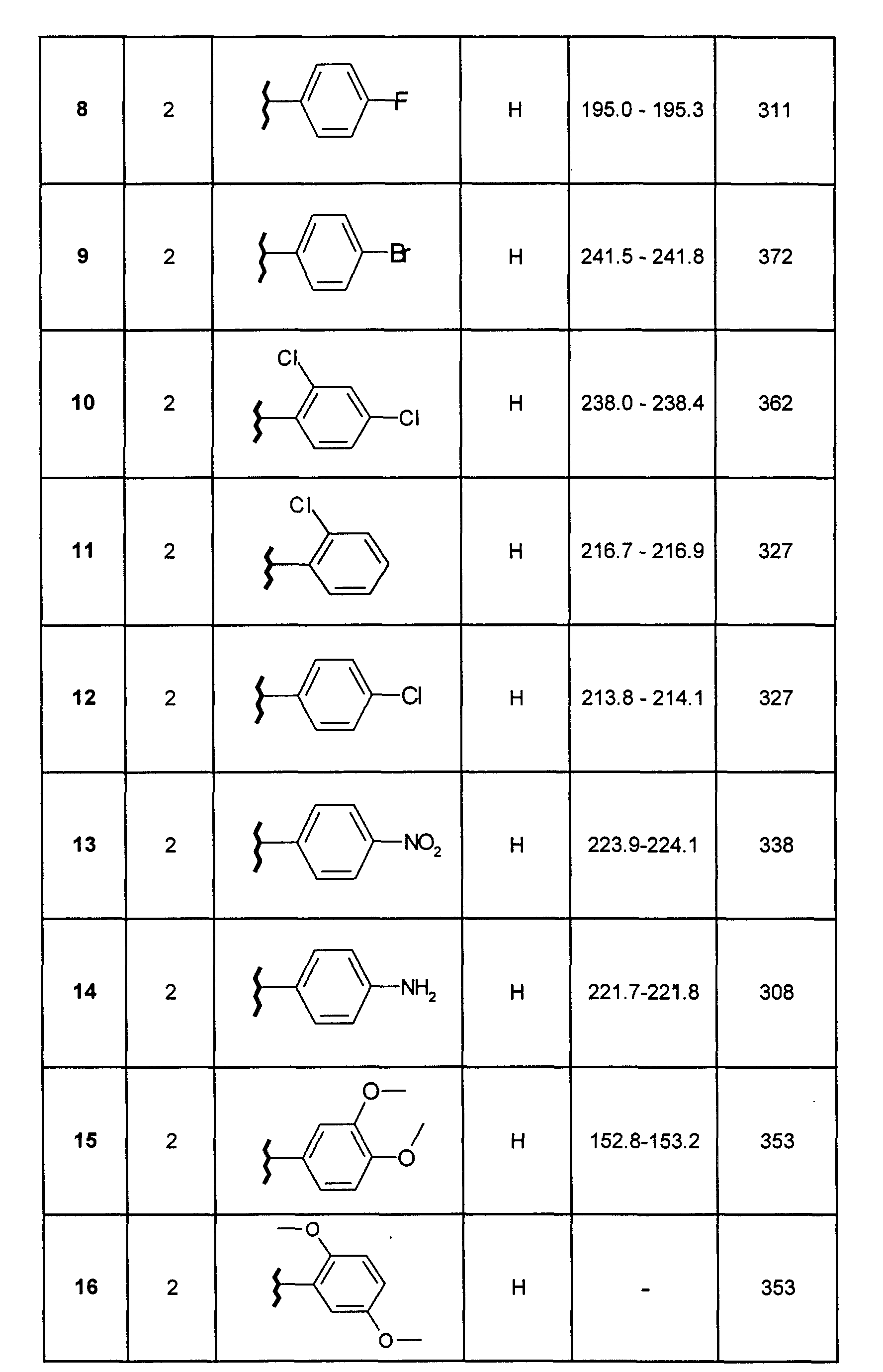

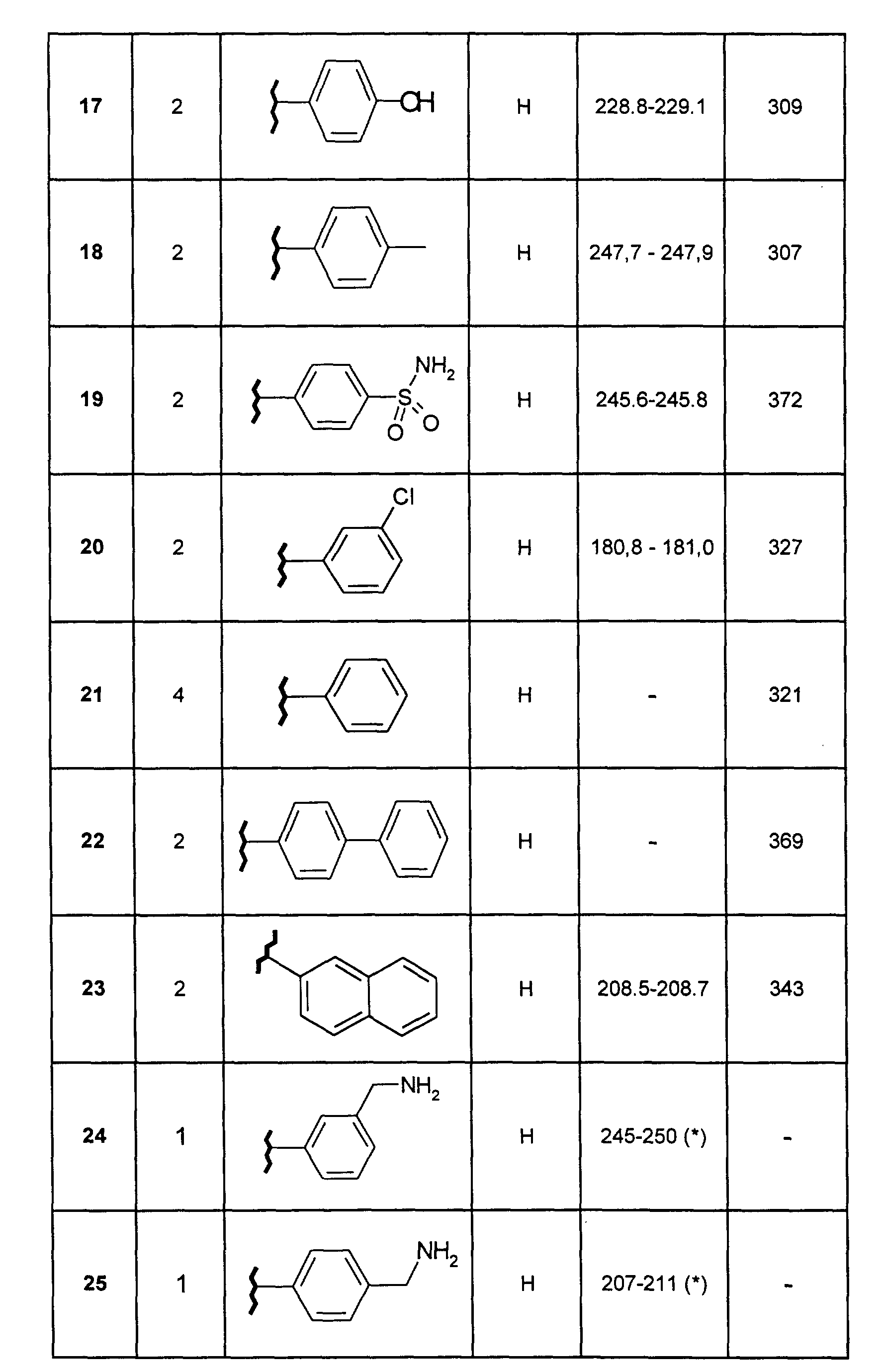

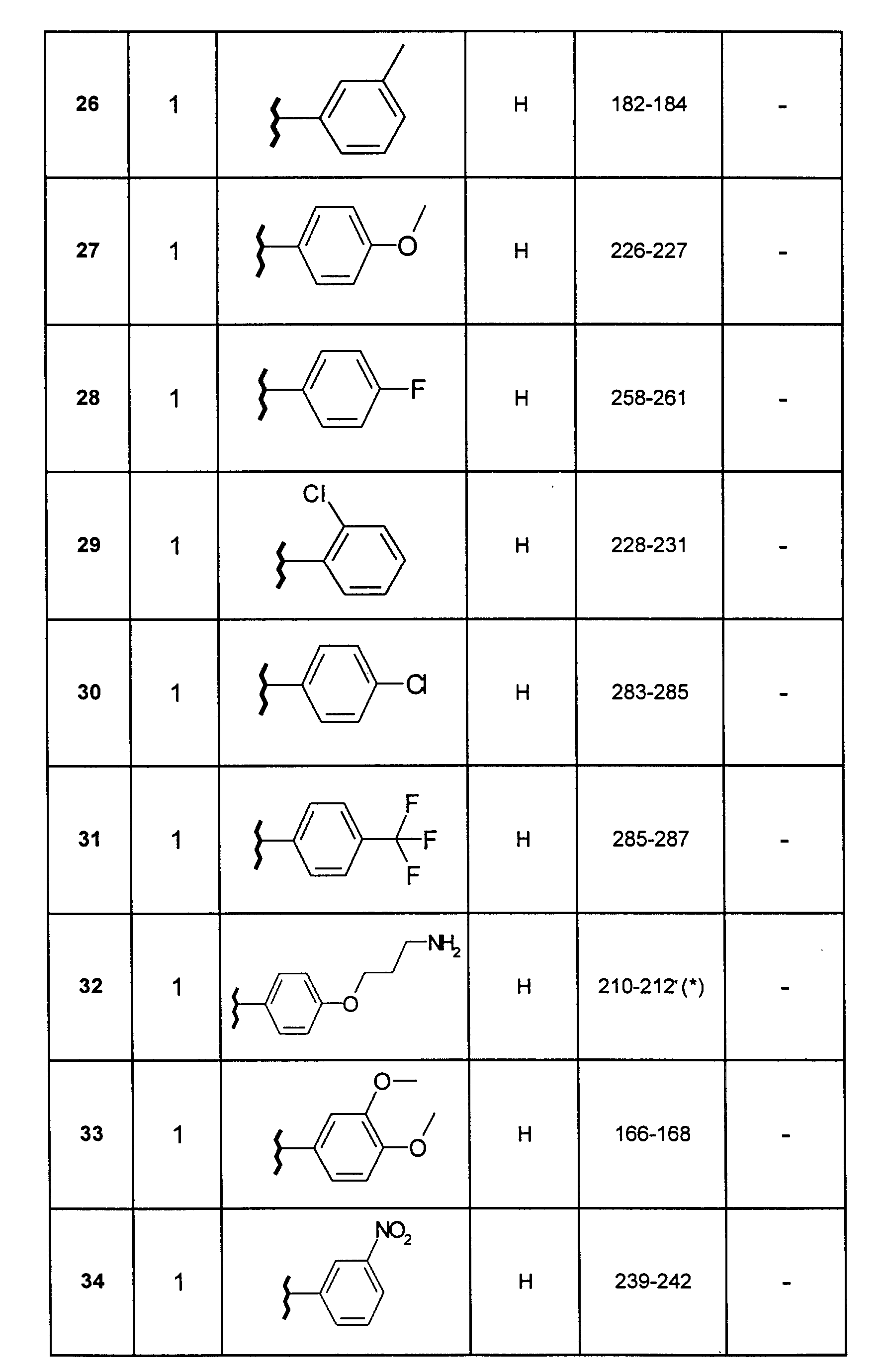

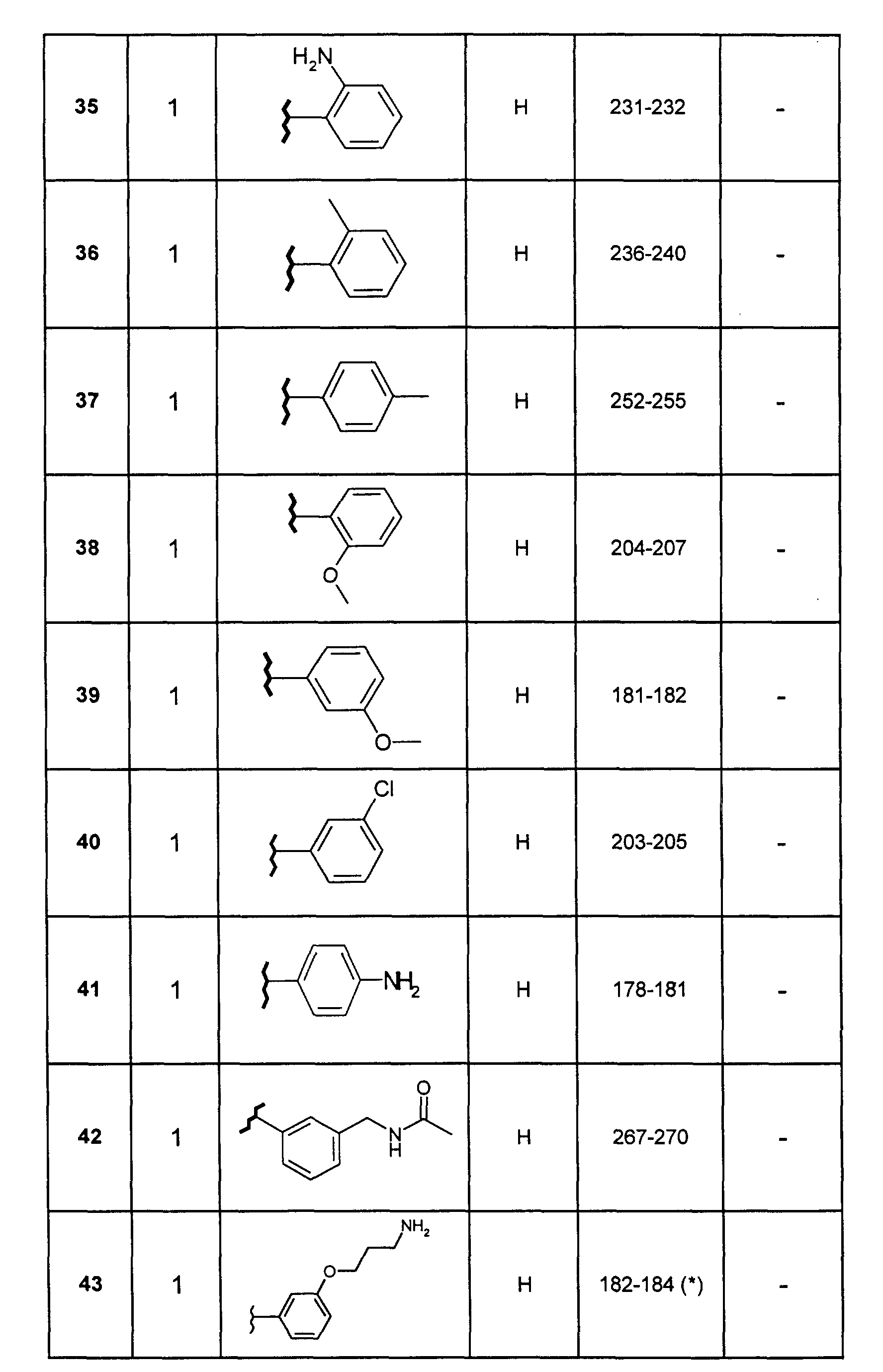

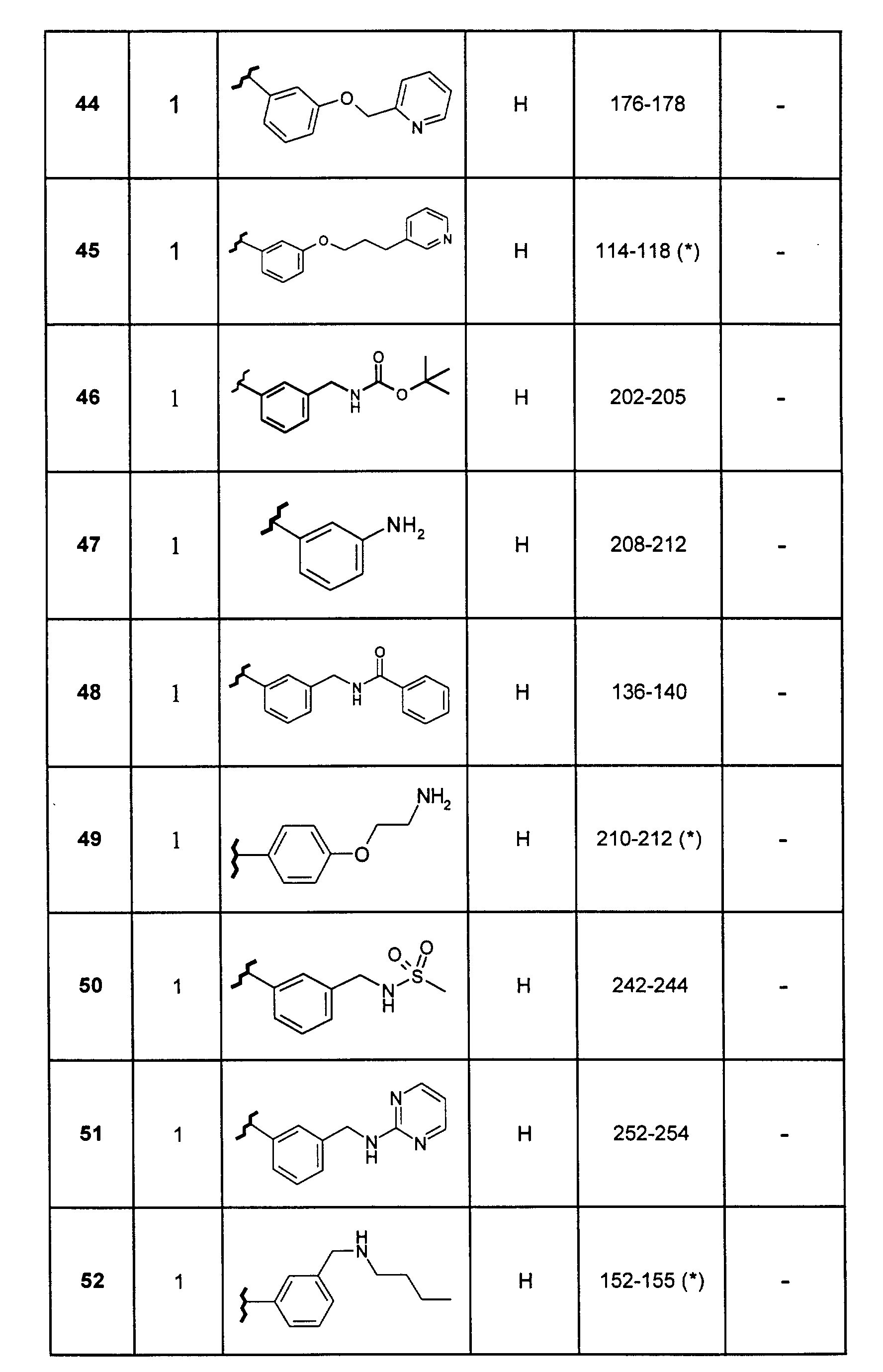

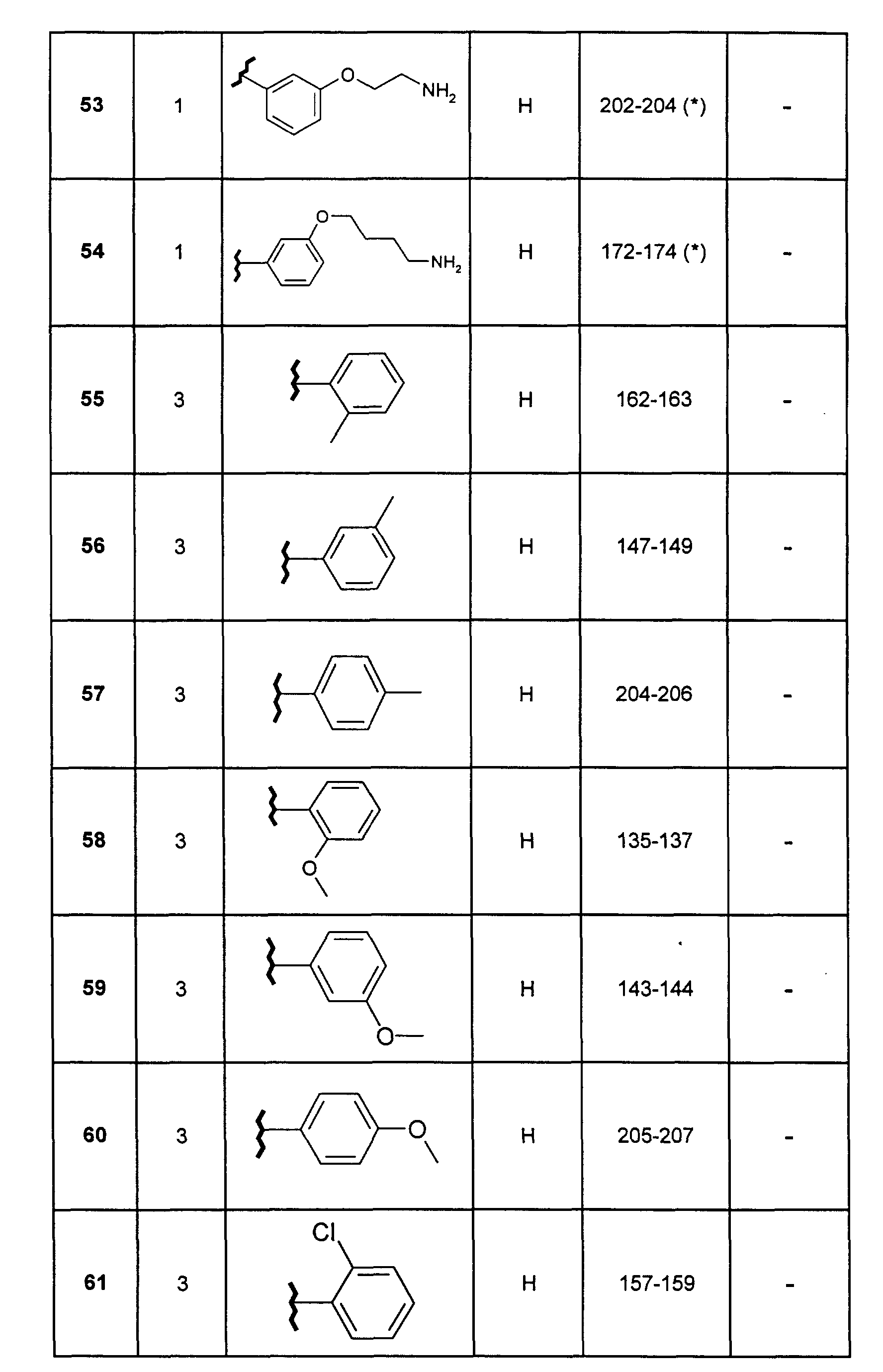

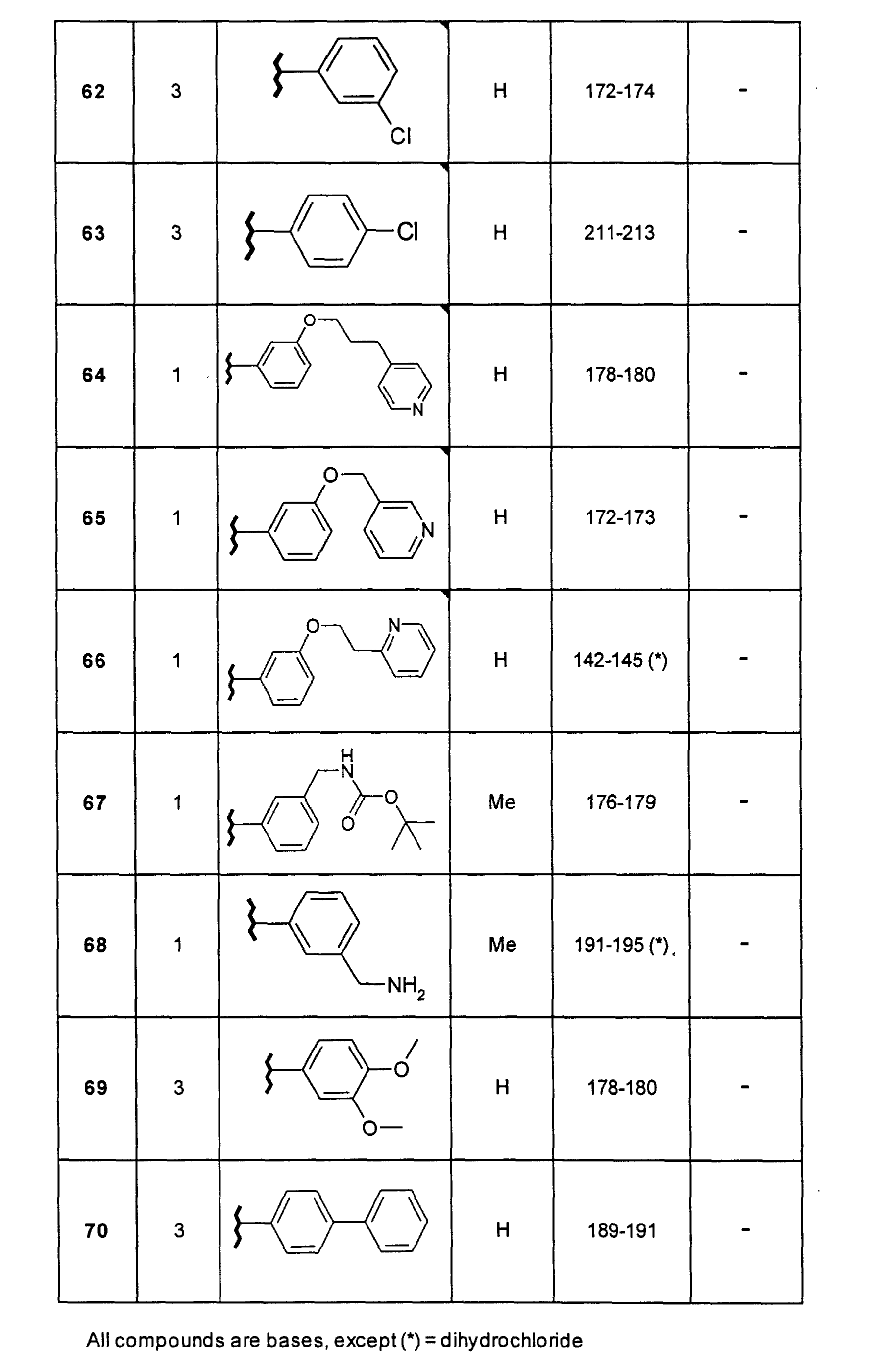

### Test Example: Inhibitory activity of the medicament of the present invention against GSK3β:

Two different protocols can be used.

In a first protocol : 7.5 µM of prephosphorylated GS1 peptide and 10 µM ATP (containing 300,000 cpm of 33P-ATP) were incubated in 25 mM Tris-HCI, pH 7.5, 0.6 mM DTT, 6 mM MgCl₂, 0.6 mM EGTA, 0.05 mg/ml BSA buffer for 1 hour at room temperature in the presence of GSK3beta (total reaction volume : 100 microliters).

In a second protocol : 4.1 µM of prephosphorylated GS1 peptide and 42 µM ATP (containing 260,000 cpm 33P-ATP) were incubated in 80 mM Mes-NaOH, pH 6.5, 1 mM Mg acetate, 0.5 mM EGTA, 5 mM 2-mercaptoethanol, 0.02% Tween 20, 10% glycerol buffer for 2 hours at room temperature in the presence of GSK3beta. Inhibitors were solubilised in DMSO (final solvent concentration in the reaction medium, 1%).

The reaction was stopped with 100 microliters of a solution made of 25 g polyphosphoric acid (85% P₂O₅), 126 ml 85% H₃PO₄, H₂O to 500 ml and then diluted to 1 :100 before use. An aliquot of the reaction mixture was then transferred to Whatman P81 cation exchange filters and rinsed with the solution described above. Incorporated 33P radioactivity was determined by liquid scintillation spectrometry.
The phosphorylated GS-1 peptide had the following sequence :

NH2-YRRAAVPPSPSLSRHSSPHQS(P)EDEE-COOH.

The GSK3β inhibitory activity of the compounds of the present invention are expressed in IC₅₀, and, as an illustration the range of IC₅₀'s for the compounds in table 1 is between 0,01 to 10 micromolar concentrations.

### Formulation Example

### (1) Tablets

The ingredients below were mixed by an ordinary method and compressed by using a conventional apparatus.

| | |
|---|---|
| Compound of Example 1 | 30 mg |
| Crystalline cellulose | 60 mg |
| Corn starch | 100 mg |
| Lactose | 200 mg |
| Magnesium stearate | 4 mg |

### (2) Soft capsules

The ingredients below were mixed by an ordinary method and filled in soft capsules.

| | |
|---|---|
| Compound of Example 1 | 30 mg |
| Olive oil | 300 mg |
| Lecithin | 20 mg |

### (1) Parenteral preparations

The ingredients below were mixed by an ordinary method to prepare injections contained in a 1 ml ampoule.

| | |
|---|---|
| Compound of Example 1 | 3 mg |
| Sodium chloride | 4 mg |
| Distilled water for injection | 1 ml |

### Industrial Applicability

The compounds of the present invention have GSK3β inhibitory activity and are useful as an active ingredient of a medicament for preventive and/or therapeutic treatment of neurodegenerative diseases caused by abnormal activity of GSK3β.

## Claims

1. A pyrimidone derivative represented by formula (I) or a salt thereof, or a solvate thereof or a hydrate thereof: 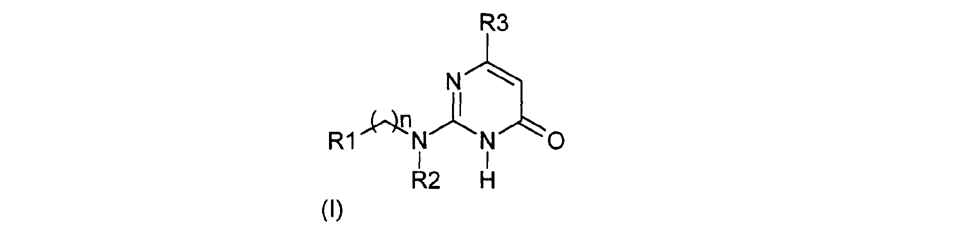 wherein:
when n represents 1 to 5, then R1 represents an unsubstituted napht-1-yl group, an unsubstituted napht-2-yl group or a C_{6,10} aryl group substituted by 1 to 3 substituents selected from a C₁₋₆ alkyl group, halogen atom, a C₁₋₂ perhalogenated alkyl group, a C₁₋₃ halogenated alkyl group, a hydroxyl group, a C₁₋₆ alkoxy group, methylenedioxy group, a nitro, a cyano, an amino, a C₁₋₆ monoalkylamino group, a C₂₋₁₂ dialkylamino group, a (C₁₋₆-alkyl)carbonylamino group, a (C_{6,10}-aryl)carbonylamino group, a (C₁₋₆ -alkoxy)carbonylamino group, aminocarbonyl group, a (C₁₋₆ monoalkylamino)carbonyl group, a (C₂₋₁₂ dialkylamino)carbonyl group, a formyl, a C₁₋₆ alkylcarbonyl group, a (C_{6,10}-aryl)carbonyl group, a C₁₋₆ alkylsulfonyl group, a C_{6,10} arylsulfonyl group, an aminosulfonyl group, a C₁₋₆ monoalkylaminosulfonyl group, a C₂₋₁₂ dialkylaminosulfonyl group, or a phenyl group;
the C₁₋₆ alkyl groups and the C₁₋₆ alkoxy groups being optionally substituted by a halogen atom, a hydroxyl group, a C₁₋₆ alkoxy group, an amino, a C₁₋₆ monoalkylamino group, a C₂₋₁₂ dialkylamino group, a (C₁₋₆ alkyl)carbonylamino group, a (C_{6,10} aryl)carbonylamino group, a (C₁₋₆alkoxy)carbonylamino group, a C₁₋₆ alkylsulfonylamino group, a C_{6,10} arylsulfonylamino group, a phenyl group, a pyridine, a pyrimidine, or a pyrimidin-2-yl-amino,
or when n represents 4 or 5 then R1 can represent an unsubstituted phenyl group;
R2 represents a hydrogen atom or a C₁₋₆ alkyl; and
R3 represents a 2, 3 or 4-pyridyl group optionally substituted by a C₁₋₄ alkyl, group, C₁₋₄ alkoxy group or a halogen atom.

2. A pyrimidone derivative or a salt thereof, or a solvate thereof or a hydrate thereof according to claim 2, wherein R3 is an unsubstituted 4-pyridyl group.

3. A pyrimidone derivative or a salt thereof, or a solvate thereof or a hydrate thereof according to claim 2, wherein n represents 1 to 4

4. A pyrimidone derivative which is selected from the group consisting of:
2-[[(3,4-dimethoxyphenyl)methyl]amino]-6-pyridin-4-ylpyrimidin-4-(1H)-one,
2-[[2-(3,4-dimethoxyphenyl)ethyl]methylamino]-6-pyridin-4-ylpyrimidin-4-(1H)-one,
2-[[2-(4-methoxyphenyl)ethyl]amino]-6-pyridin-4-ylpyrimidin-4-(1H)-one,
2-[[2-(3-methoxyphenyl)ethyl]amino]-6-pyridin-4-ylpyrimidin-4-(1H)-one,
2-[[2-(2-methoxyphenyl)ethyl]amino]-6-pyridin-4-ylpyrimidin-4-(1H)-one,
2-[[2-(2-fluorophenyl)ethyl]amino]-6-pyridin-4-ylpyrimidin-4-(1H)-one,
2-[[2-(3-fluorophenyl)ethyl]amino]-6-pyridin-4-ylpyrimidin-4-(1H)-one,
2-[[2-(4-fluorophenyl)ethyl]amino]-6-pyridin-4-ylpyrimidin-4-(1H)-one,
2-[[2-(4-bromophenyl)ethyl]amino]-6-pyridin-4-ylpyrimidin-4-(1H)-one,
2-[[2-(2,4-dichlorophenyl)ethyl]amino]-6-pyridin-4-ylpyrimidin-4-(1H)-one,
2-[[2-(2-chlorophenyl)ethyl]amino]-6-pyridin-4-ylpyrimidin-4-(1H)-one,
2-[[2-(4-chlorophenyl)ethyl]amino]-6-pyridin-4-ylpyrimidin-4-(1H)-one,
2-[[2-(4-nitrophenyl)ethyl]amino]-6-pyridin-4-ylpyrimidin-4-(1H)-one,
2-[[2-(4-aminophenyl)ethyl]amino]-6-pyridin-4-ylpyrimidin-4-(1H)-one,
2-[[2-(3,4-dimethoxyphenyl)ethyl]amino]-6-pyridin-4-ylpyrimidin-4-(1H)-one,
2-[[2-(2,5-dimethoxyphenyl)ethyl]amino]-6-pyridin-4-ylpyrimidin-4-(1H)-one,
2-[[2-(4-hydroxyphenyl)ethyl]amino]-6-pyridin-4-ylpyrimidin-4-(1H)-one,
2-[[2-(4-methylphenyl)ethyl]amino]-6-pyridin-4-ylpyrimidin-4-(1H)-one,
2-[[2-(4-aminosulfonylphenyl)ethyl]amino]-6-pyridin-4-ylpyrimidin-4-(1H)-one,
2-[[2-(3-chlorophenyl)ethyl]amino]-6-pyridin-4-ylpyrimidin-4-(1H)-one,
2-[[4-(phenyl)butyl]amino]-6-pyridin-4-ylpyrimidin-4-(1H)-one,
2-[[2-(4-phenylphenyl)ethyl]amino]-6-pyridin-4-ylpyrimidin-4-(1H)-one,
2-[[2-(2-naphthyl)ethyl]amino]-6-pyridin-4-ylpyrimidin-4-(1H)-one,
2-[[[3-(aminomethyl)phenyl]methyl]amino]-6-pyridin-4-ylpyrimidin-4-(1H)-one,
2-[[[4-(aminomethyl)phenyl]methyl]amino]-6-pyridin-4-ylpyrimidin-4-(1H)-one,
2-[[(3-methylphenyl)methyl]amino]-6-pyridin-4-ylpyrimidin-4-(1H)-one,
2-[[(4-methoxyphenyl)methyl]amino]-6-pyridin-4-ylpyrimidin-4-(1H)-one,
2-[[(4-fluorophenyl)methyl]amino]-6-pyridin-4-ylpyrimidin-4-(1H)-one,
2-[[(2-chlorophenyl)methyl]amino]-6-pyridin-4-ylpyrimidin-4*-*(1H)-one,
2-[[(4-chlorophenyl)methyl]amino]-6-pyridin-4-ylpyrimidin-4-(1H)-one,
2-[[[4-(trifluoromethyl)phenyl]methyl]amino]-6-pyridin-4-ylpyrimidin-4-(1H)-one,
2-[[[4-(3-aminopropoxy)phenyl]methyl]amino]-6-pyridin-4-ylpyrimidin-4-(1H)-one,
2-[[(3,4-dimethoxyphenyl)methyl]amino]-6-pyridin-4-ylpyrimidin-4-(1H)-one,
2-[[(3-nitrophenyl)methyl]amino]-6-pyridin-4-ylpyrimidin-4-(1H)-one,
2-[[(2-aminophenyl)methyl]amino]-6-pyridin-4-ylpyrimidin-4-(1H)-one,
2-[[(2-methylphenyl)methyl]amino]-6-pyridin-4-ylpyrimidin-4-(1H)-one,
2-[[(4-methylphenyl)methyl]amino]-6-pyridin-4-ylpyrimidin-4-(1H)-one,
2-[[(2-methoxyphenyl)methyl]amino]-6-pyridin-4-ylpyrimidin-4-(1H)-one,
2-[[(3-methoxyphenyl)methyl]amino]-6-pyridin-4-ylpyrimidin-4-(1H)-one,
2-[[(3-chlorophenyl)methyl]amino]-6-pyridin-4-ylpyrimidin-4-(1H)-one,
2-[[(4-aminophenyl)methyl]amino]-6-pyridin-4-ylpyrimidin-4-(1H)-one,
2-[[[3-(acetamidomethyl)phenyl]methyl]amino]-6-pyridin-4-ylpyrimidin-4-(1H)-one,
2-[[[3-(3-aminopropoxy)phenyl]methyl]amino]-6-pyridin-4-ylpyrimidin-4-(1H)-one,
2-[[[3-[(pyridin-2-yl)methoxy]phenyl]methyl]amino]-6-pyridin-4-ylpyrimidin-4-(1H)one,
2-[[[3-[3-(pyridin-3-yl)propoxy]phenyl]methyl]amino]-6-pyridin-4-ylpyrimidin-4-(1H)-one,
2-[[[3-(*tert*-butyloxycarbonylaminomethyl)phenyl]methyl]amino]-6-pyridin-4-ylpyrimidin-4-(1H)-one,
2-[[(3-aminophenyl)methyl]amino]-6-pyridin-4-ylpyrimidin-4-(1H)-one,
2-[[[3-(benzoylaminomethyl)phenyl]methyl]amino]-6-pyridin-4-ylpyrimidin-4-(1H)-one,
2-[[[4-(2-aminoethoxy)phenyl]methyl]amino]-6-pyridin-4-ylpyrimidin-4-(1H)-one,
2-[[[3-(methanesulfonylaminomethyl)phenyl]methyl]amino]-6-pyridin-4-ylpyrimidin-4-(1H)-one,
2-[[[3-[(pyrimidin-2-yl)aminomethyl]phenyl]methyl]amino]-6-pyridin-4-ylpyrimidin-4-(1H)-one,
2-[[[3-(n-butylaminomethyl)phenyl]methyl]amino]-6-pyridin-4-ylpyrimidin-4-(1H)one,
2-[[[3-(2-aminoethoxy)phenyl]methyl]amino]-6-pyridin-4-ylpyrimidin-4-(1H)-one,
2-[[[3-(4-aminobutoxy)phenyl]methyl]amino]-6-pyridin-4-ylpyrimidin-4-(1H)-one,
2-[[3-(2-methylphenyl)propyl]amino]-6-pyridin-4-ylpyrimidin-4-(1H)-one,
2-[[3-(3-methylphenyl)propyl]amino]-6-pyridin-4-ylpyrimidin-4-(1H)-one,
2-[[3-()propyl]amino]-6-pyridin-4-ylpyrimidin-4-(1H)-one,
2-[[3-(2-methoxyphenyl)propyl]amino]-6-pyridin-4-ylpyrimidin-4-(1H)-one,
2-[[3-(3-methoxyphenyl)propyl]amino]-6-pyridin-4-ylpyrimidin-4-(1H)-one,
2-[[3-(4-methoxyphenyl)propyl]amino]-6-pyridin-4-ylpyrimidin-4-(1H)-one,
2-[[3-(2-chlorophenyl)propyl]amino]-6-pyridin-4-ylpyrimidin-4-(1H)-one,
2-[[3-(3-chlorophenyl)propyl]amino]-6-pyridin-4-ylpyrimidin-4-(1H)-one,
2-[[3-(4-chlorophenyl)propyl]amino]-6-pyridin-4-ylpyrimidin-4-(1H)-one,
2-[[[3-[3-(pyridin-4-yl)propoxy]phenyl]methyl]amino]-6-pyridin-4-ylpyrimidin-4-(1H)one,
2-[[[3-[(pyridin-3-yl)methoxy]phenyl]methyl]amino]-6-pyridin-4-ylpyrimidin-4-(1H)-one,
2-[[[3-[2-(pyridin-2-yl)ethoxy]phenyl]methyl]amino]-6-pyridin-4-ylpyrimidin-4-(1H)one,
2-[[[3-(*tert*-butyloxycarbonylaminomethyl)phenyl]methyl]methylamino]-6-pyridin-4-ylpyrimidin-4-(1H)-one,
2-[[[3-(methylamino)phenyl]methyl]methylamino]-6-pyridin-4-ylpyrimidin-4-(1H)-one,
2-[[3-(3,4-dimethoxyphenyl)]propyl]amino]-6-pyridin-4-ylpyrimidin-4-(1H)-one and
2-[[3-(4-phenylphenyl)]propyl]amino]-6-pyridin-4-ylpyrimidin-4-(1H)-one
or a salt thereof, or a solvate thereof or a hydrate thereof.

5. A medicament comprising as an active ingredient a substance selected from the group consisting of a pyrimidone derivative according to claim 1.

6. A GSK3β inhibitor selected from the group of a pyrimidone derivative according to claim 1.

7. Use of a compound according to claim 1 for the preparation of a medicament for preventive and/or therapeutic treatment of a disease caused by abnormal GSK3β activity.

8. Use of a compound according to claims 1 to 4 for the preparation of a medicament for preventive and/or therapeutic treatment of a neurodegenerative disease.

9. Use of a compound according to claim 8, wherein the disease is selected from the group consisting of Alzheimer's disease, Parkinson's disease, frontoparietal dementia, corticobasal degeneration, Pick's disease, cerebrovascular accidents, brain and spinal cord trauma and peripheral neuropathies.
